# EUROPÄISCHE PATENTANMELDUNG

(11) **EP 1 004 368 A2**
(43) Veröffentlichungstag der Anmeldung: **31.05.2000**
(21) Anmeldenummer: 99123194.5
(22) Anmeldetag: 24.11.1999
(51) Int. Cl.: B09B 3/00, F02C 6/00, A61L 11/00

(54) **Verfahren und Anlage zur Stoffbehandlung und/oder Gasgewinnung insbesondere durch heisse Gase von Verbrennungsmotoren oder Gasturbinen**

(30) Priorität: 27.11.1998 DE 19854871
(71) Anmelder: Schmalz, Reinhold, 82008 Unterhaching (DE)
(72) Erfinder: Schmalz, Reinhold, 82008 Unterhaching (DE)

(57) **Zusammenfassung**

Ziel der Erfindung ist es, daß fast die gesamte mechanische als auch thermische Nutzenergie der V-Motoren einschließlich deren Schallwellen und keimtötenden Abgase sowie Kühlmittel zur Stofftrennung /-behandlung, Sterilisation und / oder Frischgasgewinnung verwendet werden.

**Beispiel**: Im Leer- und Lastbetrieb werden die heißen Gase, vorzugsweise von V-Motoren, durch die Anlage, die auch als 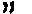 Auspuffanlage" dient, von der Materialeingabe bis zu den Restmaterial-Aufnahmebehälter, zwecks Sterilisation des Materials sowie Verwertung und energetischer Verbrennung der Restgase, ggf. im Kreislauf dem V-Motor aufbereitet zugeführt, wobei der Abgasdruck - falls notwendig - einen Kolben im Auspuffrohr (einstellbares Überdruckventil) soweit axial verschiebt, daß Abgase ins Freie gelangen. Die heißen / keimtötenden Abgase schießen druckvoll in den Materialeingabebehälter, treiben dort eine Turbine zwecks Materialzerkleinerung und -transport an, schmelzen die Kunststoffverpackungen auf, lassen das Material und dessen Festigkeit schrumpfen, Glasgefäße zerbrechen, erzeugen durch Verschwelung Frischgase, sterilisieren, drücken bei Bedarf den sterilisierten Materialrest (ca. 10% des ursprünglichen Volumens) durch ein Rohr in einen Material-/Gas-Sammelbehälter. Wegen Sauerstoffmangel in der gesamten Anlage - außer V-Motor - können sich die durch Verschwelung entstandenen Frischgase nicht entzünden. Falls vorrangig die Separierung zum Beispiel von Quecksilber oder Nutzgasen zum Beispiel aus Biomaterial erzielt werden soll, werden die Gase einem Gas- und Stoffabscheider (u.a. Abgaszentrifuge) zugeführt. Der V-Motor erzeugt während des Verfahrensprozesses die notwendige thermische, mechanische, chemische, elektrische Energie und Druckluft, so daß ideale Bedingungen für den mobilen / stationären Betrieb der Anlage geschaffen wurden.

## Beschreibung

Ziel der Erfindung ist es, die gesamte mechanische als auch thermische Nutzenergie der V-Motoren einschließlich deren Schallwellen und keimtötenden Abgase sowie Kühlmittel optimal zur Stofftrennung/-behandlung, Sterilisation und/oder Frischgasgewinnung sowie deren wirtschaftliche Verwertung zu verwenden.

Der Verfahrensprozeß kann separat und kombiniert erfolgen durch:
- - Heiße, toxische Gase: (vorzugsweise von V-Motoren, die eine Abgasturbine antreiben),
- - Mechanischen Druck und Reibung: (u.a.Abgasturbine = Material-Zerkleinerer/-Förderer, Abgasreiniger durch Fliehkraft),
- - Gasüber- / -Unterdruck: (V-Motoren und/oder Turbolader aller Art, Druckluft),
- - Chemische Technologie: (Ab- und Friachgase oder bei Bedarf sonstige Mittel),
- - Fliehkraft:
und ggf. in Kombination mit
- Mikrowellen-/Schallwellenenergie,
- Bestrahlungen
- Magnetfelder
- Wirbelstromabscheider
- Elektrische Energie sowie
   Elektrostatische Maßnahmen. Hierbei können Flüssigkeiten und oder Gase als Durchlaufkühlmittel für V-Motoren erhitzt, entkeimt bzw. vergast/-dampft und zweckentsprechend - auch im Kreislauf - aufbereitet werden.

Aufgabe der Erfindung war es, ein Verfahren und eine Anlage zu schaffen, die betriebssicher
- die Entsorgungskosten,
- den Energiebedarf,
- die Umweltbelastung,
- die Luftschadstoffe,
- den Geräuschpegel,
- die ozonbildenden Stickoxide,
- die CO₂-Belastung,
- den Ressourcenverbrauch
- die Geruchsbelästigung
- die Treibhausgase
- die Klima- und Ozonschichtbelastung
drastisch senkt. Ferner den Naturhaushalt schont und eine umweltgerechte Mobilität ermöglicht. Zwecks Erreichung des Klimaziels sind eine effizientere Umwandlung und Anwendung von Energie dringend notwendig. Nutzen: Zusätzliche Arbeitsplätze, weniger Kosten, mehr Klimaschutz. Strom sollte nicht für Zwecke verwendet werden, für die es bessere Alternativen gibt. So ist das Heizen mit Strom Energievergeudung und verursacht besonders hohe CO₂-Emissionen. Der produktintegrierte Umweltschutz erzeugt, wie hier geplant, wenig Restabfall, der weiterverwendet werden kann, ohne daß gesundheitsschädliche Keime / Stoffe die Umwelt belasten. Die Deponiekosten für Sonder- und Hausmüll werden in den nächsten Jahren um 100 bis 200% ansteigen. Dies begünstigt die Entwicklung, den Bau und den wirtschaftlichen Betrieb der relevanten Anlagen. Bei dem nachstehend vorgestellten Verfahren handelt es sich um eine besonders saubere und lärmarme Technologie.

Das **Verfahren** und die dazu erforderliche **Anlage** wird wie folgt beschrieben: **Beispie**l: Im Leer- und Lastbetrieb werden die heißen und **keimtötenden** Abgase, vorzugsweise von V-Motoren durch die Anlage, die auch als :"Auspuffanlage" dient, von der Materialeingabe bis in den Aufnahmebehälter des behandelnden Materials, geführt. Somit erreicht die Anlage bereits im **Leerbetrieb** den zur Sterilisation erforderlichen Betriebszustand, wobei der Abgasdruck einen Kolben im Auspuffrohr soweit axial verschiebt, daß die Abgase ins Freie gelangen (Überdrucksicherung). Eine Abgasturbine (Rohrturbine) reinigt durch Fliehkraft und evtl. durch Oxidation die Abgase; Rußpartikel, Asche etc. lagern sich ab.

Im **Lastbetrieb** wird aus dem Aufnahmebehälter für das behandelte Material das Abgas und aus dem Müll gewonnene Frischgas über Vorfilter zwecks Verwertung / Entsorgung und gegebenenfalls energetischer Verbrennung dem V-Motor, etc. zugeführt. Normalerweise werden die heißen Abgase und Schallwellenenergie vom Motorauslaß direkt in den Materialeingabebehälter gedrückt, damit das Material und dessen Festigkeit drastisch schrumpft sowie der Eingabebehälter permanent sterilisiert wird. Falls erforderlich, wird das Material zusätzlich mechanisch so zerkleinert, daß die heißen / keimtötenden Gase das Granulat gänzlich sicher über 133°C erhitzen und zusätzlich über den dadurch entstehenden Wasserdampf schnell sterilisieren. Wegen Sauerstoffmangel in der gesamten Anlage können sich die durch die Verschwelung des Materials entstandenen Frischgase nicht entzünden; diese gelangen im Flußprinzip zusammen mit dem verwirbelten Granulat durch ein Rohr in einen gasdichten Transportbehälter. Das sterilisierte Material / Granulat und die Asche saugen den überschüssigen Wasserdampf bis zum Ende des Verfahrensprozesses weitgehend auf, die Gastemperatur sinkt weiter ab. Zuvor werden die heißen Gase durch die starke Erhitzung und teilweise Verdampfung / Vergasung des Materials schon enorm abgekühlt. Aus dem Granulat-Transportbehälter werden die gemixten Ab- und Frischgase über Vorfilter einem Verbrennungsmotor, etc. zwecks Entsorgung und energetischer Verbrennung zugeführt, dabei wird dem Gas weiter Wärme entzogen (Ladeluftkühlung) und soweit (wenn erforderlich durch Sauerstoff¹) aufbereitet, daß eine störungsfreie Verbrennung erfolgen kann. Zweckentsprechend können die Gase auch ganz oder teilweise im Kreislauf durch den Materialeingabebehälter geleitet und verwertet werden.
¹ 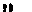 Lamda-Sonde"

Falls vorrangig die Rückgewinnung von Quecksilber etc. erzielt werden soll, werden die sehr heißen quecksilberhaltigen Gase u.a. einem Gas- und Stoffabscheider (ge gebenenfalls. Gaszentrifuge) zugeführt. Ebenfalls gilt dies für die Gewinnung von Nutzgasen, z.B. aus Biomaterial.

Das Ziel wird durch die gekoppelte Erzeugung von Kraft, Wärme, Hitze, Kälte, Strom, Druckluft und chemische Energie erreicht.

Möglich ist auch, daß die Ab- und Frischgase aus der Anlage einem zweiten V-Motor, z.B. Stromaggregat oder Gasbrenner bzw. Stirlingmotor zur Verwertung zugeführt werden. V-Motoren mit der Werkstoff-Kombination Kohlenstoff / Keramik, z.B. Kohlenstoff-Kolben / Keramik-Laufbuchsen und -Ventilen erhöhen im Gas- / Dampfbetrieb den Gesamtwirkungsgrad erheblich.

V-Motoren erzeugen während des Verfahrensprozesses die notwendige thermische, chemische, mechanische, elektrische Energie und Druckluft, so daß ideale Bedingungen für den mobilen / stationären Betrieb der Anlage geschaffen wurden. Hochverdichtete Gase reduzieren den Bedarf an Prozeßenergie. Gerade im Zuge der weltweit fortschreitenden Energieverteuerung und Umweltverschmutzung ist die vorgestellte Anlage genial, denn die benötigte Verfahrensenergie fällt überwiegend als Nebenprodukt an. Verstärkt wird dieser Effekt noch dadurch, daß das Materialvolumen von 100% auf unter 10% durch das beschriebene Verfahren sowie die Transportkosten minimiert werden. Die rationelle Energiegewinnung und -verwendung erreicht eine bisher noch nicht erzielte Wirtschaftlichkeit.

Bei Bedarf kann fast die gesamte "thermische Energie" durch Kapselung des Motors in die gasdichte Anlage eingespeist werden. Für Verbrennungsmotoren stehen inzwischen ausgereifte Kohlenstoff-Kolben zur Verfügung, die höhere Gastemperaturen und aggressive Frisch- und Abgase gut vertragen. Im Vergleich mit Alu-Kolben bietet der Kohlenstoff-Kolben noch weitere Vorteile, auf die hier aber nicht näher einzugehen ist.

Die Kapazität des Materialbehandlungsbehälters ist so zu bemessen, daß eine betriebswirtschaftlich sinnvolle Charge ohne Unterbrechung behandelt werden kann. Beide vorgenannte Behälter dienen auch als Gas- und Druckspeicher. Ein Füllstandsanzeiger meldet rechtzeitig, wann der Material- / Granulat-Behälter voll und zu wechseln ist.

Befüllt wird der Material-Eingabebeälter:
a) Direkt oder
b) Das abdichtende Material wird durch ein Rohr mit druckdichter Schleuse (z.B. axial verschiebbarer Kolben) gedrückt oder
c) Über eine druckdichte Vorrichtung, ähnlich einer modernen Müllschluckanlage, die auch als Doppelrohr ausgeführt sein kann, wobei die walzenförmige Halbschale nach der Befüllung um 180 Grad verdreht wird, damit das Material in den Behälter fällt.
d) Während des Füllvorgangs können die Gase abgesaugt, die Abgaszufuhr unterbrochen und / oder der V-Motor per Fernbedienung abgestellt werden.
e) Durch Zahnradwalzen, die das Material einziehen (siehe Blatt 6).

Nach der Eingabe des Materials (handelsübliche Einweg-Sammelbehälter aus Kunststoff, Inhalt: meistens Kunststoffe, Papier, Verbandsstoffe, Spritzen, Kanülen, Glasgefäße etc.) wird der Eingabebehälter gasdicht verschlossen, der V-Motor gestartet; die heißen und keimtötenden Motor-Abgase treiben eine Turbine (am Boden im Materialeingabebehälter an der volle Gasdruck wirkt durch eine Rohrturbine an speziellen Gasaustrittsöffnungen so nach außen, daß die dort angebrachten Schneiden das Material thermisch / mechanisch zerkleinern und dieses in das Material- und Gasabführrohr fördern. Die Rohrturbine kann zusätzlich auch anderweitig, z.B. durch Druckluft angetrieben werden und in Kegelform (Spänetransport sowie unterschiedliche Hebelkräfte und Spanleistung) ausgeführt sein. Die aus der Gasturbine ausströmenden heißen Gase schmelzen die Kunststoffpackungen auf, lassen das Material und dessen Festigkeit schrumpfen, Glasgefäße zerbrechen, sterilisieren, erzeugen durch Verschwelung des Materials Frischgase, drücken das sterilisierte Material und den Gasmix durch ein Rohr, das je nach Bedarf eben, ganz leicht schräg nach oben oder unten, in den Material- / Gas-Sammelbehälter (so tief wie möglich gelegt) verläuft. Somit kann die Anlage alle gängigen Gebinde bei jeder Wetterlage und dynamischen Belastungen im Straßenverkehr behandeln.

Zwischen Materialeingabe-Behälter und Restmaterial-Aufnahmebehälter können ein oder mehrere Siebe das behandelte Material sortieren; so fallen Asche, Glassplitter und Metallteile etc. durch die Siebe (z.B. im Boden des Material-Eingabebehälters angeordnet), währenddessen der Gasstrom (> 0,3 m³/sek) leichte Materialschnitzel in den Aufnahmebehälter (2 Kammern) bläst. In die vordere Kammer fallen das Granulat, in die hintere Kammer die leichten Schnitzel. Eine schräge Anordnung der Siebe bzw. mit Gefälle erhöht deren Leistung (ggf Siebtrommel / Drehrohrsieb, Windsichter / Magnetabscheider verwenden).

Bei anderen **Varianten** kann das Material, in Kombination mit vorgenannten Möglichkeiten, mechanisch zerkleinert, inaktiviert und gefördert werden durch:
- Schneid- und Förderschnecken, mit / ohne Welle, im Rohr und / oder auf Verschleiß-/ Schneidschienen, gelagert, mit / ohne unterschiedlichen Steigungswinkein; Spitze und Führungsrohr zumindest teilweise konisch, wobei hier die Schneiden die Rohrinnenwand nicht berühren (Vor- und Rücklauf), können analog, wie nachstehend unter 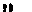 Zahnradpaar" dargelegt, durch den Einsatz von Starkstrom gegebenenfalls bessere Ergebnisse erzielen.. Durch den Abgasdruck erfolgt eine optimierte Lagerung und das Material wird zusätzlich umgewirbelt sowie transportiert.
   Im Sonderfall kann die Lagerung auch durch runde, offene Lagerschalen (wie ein Speichenrad, jedoch nur mit einem Umfang von ca. 300 Winkelgrade, an der Rohrinnenwand abstützend, unten ca. 60 Winkelgrade offen, damit der Durchfluß nicht behindert wird, die Lagerung erfolgt in der Lagernabe des Speichenrades) geschehen.
- Zahnradpaar(e) (schräg- oder geradeverzahnt), Schraubenwalzen, das Material wird eingezogen und mit hohem Druck durch die Zähne zerkleinert (gequetscht) und gefördert; es bilden sich relativ trockenes Granulat und Flüssigkeiten, diese und Feststoffe lassen sich dann leicht trennen sowie verarbeiten; Keime werden durch Druck, Reibung und falls erforderlich elektrischer Energie sowie Ozon zerstört. Die Zähne der Zahnradwalzen leiten den Starkstrom durch das zu behandelnde Material von Zahnrad zu Zahnrad mit Schockwellen und Stromschlägen, wobei Ozon entsteht, das auch Mikroorganismen tötet. Die keimtötende Wirkung kann noch dadurch verstärkt werden, daß eine Zahnradwalze den Strom stärker leitet als die andere (n) Zahnradwalze(n), nämlich durch Strom, Hitze, Druck, Reibung und Ozon.

**Anwendungsbeispiele** hierfür sind 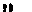 Speiseresteverwertung unter Berücksichtigung von Seuchenhygiene"; 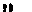 Verwertung von Abfällen der Lebensmittelindustrie"; Literatur BayFORREST, Stand Oktober 1998.

Lediglich bei der Umwandlung von Lebensmittel- und Speiseresten zu Futtermitteln wird diese Anlagenvariante nur bei Bedarf durch Abgase im Leerbetrieb sterilisiert, ansonsten geschieht dies grundsätzlich durch die keimtötenden Abgase und Schallwellen von V-Motoren bzw. Kühlmitteldampf.

Das Gas-Material-Leitungsrohr kann innen glatt, spiralförmig oder schuppenartig sein zwecks Beeinflussung der Wärme und Strömungsverhältnisse sowie der Fliehkraft. Hoher Druck = besserer Wirkungsgrad (z.B. bei der Sterilisation). Durch Verringerung der Strömungsgeschwindigkeit und der Rohrlänge ergibt sich ein geringerer Druckverlust. Im Gas- und Material-Leitungsrohr, das den Materialeingabebehälter und den Granulataufnahmebehälter verbindet, fließen die heißen, keimtötenden Gase während des Materialtransports in den Materialaufnahmebehälter weiter und vernichten sämtliche Krankheitserreger.

Je nach Verwendungszweck können die Gase, z.B. zwecks Quecksilber-Recycling oder anderer Maßnahmen separat behandelt und gespeichert werden. Quecksilber (Siedepunkt 375°C) wird durch heiße Gase von V-Motoren, (Temperatur bis ca. 800°C) gasförmig; die heißen sauerstoffarmen Gase strömen aus einer Gasdusche in ein Flüssigkeitsbecken, durch die plötzliche Abkühlung der Gase lagert sich das Quecksilber am Boden des Wasserbeckens ab. Zusätzlich wirken bei Bedarf im Flüssigkeitsbecken thermische und / oder chemische und / oder Schwach- bzw. Starkstromenergie.

Gemäß einer anderen Variante werden die Gase durch einen Wirbelstromabscheider geführt. Der durch die Behandlung des Materials entstehende Gasmix wird in der Nähe eines Rohrendes tangential eingeblasen. Die Einströmstelle unterteilt das Wirbelrohr in einen kurzen und langen Hohlraum sowie einen großen und kleinen Gasstrom. Zwischen der oder den Einströmdüsen und dem benachbarten Ende des kurzen Rohrabschnittes befindet sich eine Lochblende. Das andere Rohrende läßt sich über eine Drossel ganz oder teilweise verschließen.

Das Phänomenale am Wirbelrohr zeigt sich bei nur zum Teil geöffneter Drossel. Dann strömt kaltes Gas durch die Blendenöffnung aus und warmes über die Drossel. Die Aufteilung des einströmendes Gases in einen warmen und einen kalten Teilstrom findet im Rohr statt, in dem sich der Gesamtstrom zunächst schraubenförmig von den Düsen in Richtung Drossel bewegt. Dieser Wirbelstrom ist in seinem Kern kälter als entlang der Rohrwand. Über die Drosselstellung läßt sich das Verhältnis von kaltem zu warmem Volumenstrom regeln.

Bei der Trennung in einen kalten und einen warmen Teilstrom erfolgt auch eine Massentrennung nach Stoffdichten. Nur noch ein Bruchteil - weniger als 10% - eines kontaminierten Gasstromes muß in nachgeschalteten Anlagen gereinigt werden. Über 99% des Feststoffes lassen sich so in dem warmen Gas konzentrieren, und das unabhängig vom Partikelgehalt des zu reinigenden Gases, zum Beispiel von V-Motoren.

Das Wirbelrohr läßt sich auch im Unterdruck, zum Beispiel mit E-Turboladern nutzen. Je nach Modifizierung kann das Verfahren u.a. zur Kälteerzeugung dienen. Aus verbrannten Stoffen lassen sich Edelmetalle isolieren. Die durch die gezielte Gaseindüsung erzeugte Rotationsströmung führt zu einer Zwangssedimentation der Aschepartikel. Eine Optimierung bzw. zweckentsprechende Anpassung kann dadurch erfolgen, daß das Wirbelstromrohr dauermagnetisch ist bzw. dort elektrische und magnetische Felder wirken.

In fast allen Fällen werden anschließend die Restgase im V-Motor bei ca. 2.000°C verbrannt. Teuere Abgasfilter sind hier überflüssig Dioxine und Furane bereiten keine Probleme, sie werden in V-Motoren zerstört. Alternativ können die Gase durch V-Motoren (Saugmotoren) auch direkt abgesaugt und energetisch verwertet werden.

In einer bevorzugten Variante wird die Betriebstemperatur der Anlage nur durch die Betriebstemperatur des V-Motors geregelt. Die Materialbehandlungszeit läßt sich sicher und preisgünstig manuell oder automatisch über die Laufzeit und -leistung des V-Motors regeln.

Je nach Bedarf kann bei einer anderen Variante durch den V-Motor und zusätzlich programmiert durch Elektro- und sonstige Lader, wie z.B. Turbolader bzw. Steuerung der Abgase und mehrstufig zugeführte Verbrennungsluft, die Anlage in Normal- oder Unter- oder Überdruck mit Temperaturregelung gefahren werden. Ferner läßt sich die Behandlungszeit des Materials durch die Laufleistung des V-Motors und einen mechanischen Materialzerkleinerer und -förderer steuern. Für das beschriebene Verfahren nebst Anlage stehen robuste und vielseitig einsetzbare Gas-, Sauerstoff-, Druck-, Feuchtigkeits- und Temperatur-Sensoren (bis 800°C, kurzzeitig bis 1.000°C) zur Verfügung. Die Verfahrensparameter u.a. Temperatur, Zeit und Druck können durch ein Gerät gesteuert, überwacht, gespeichert und ausgedruckt werden.

### Abgase von Otto- (OM) und Dieselmotoren (DM)

### Zusammensetzung der Abgase in Raum-% (Beispiel)

| Abgasbestandteil | bei Leerlauf | | bei Vollast | | | |
|---|---|---|---|---|---|---|
| | | | niedere Drehzahl | | hohe Drehzahl | |
| | OM | DM | OM | DM | OM | DM |
| Kohlendioxyd CO₂ | 6,5 - 8 | 3,5 | 7 - 11 | 5,5 | 12 - 13 | 7 |
| Wasserdampf H₂O | 7 - 10 | 3,5 | 9 - 11 | 5 | 10 - 11 | 5 |
| Sauerstoff O₂ | 1 - 1,5 | 16 | 0,5 - 2 | 12 | 0,1 - 0,4 | 10 |
| Kohlenoxyd CO (*Gift!*) | 4 - 6 | bis 0,05 | 4 | 0,1 | 1 - 3 | bis 0,3 |
| Wasserstoff H₂ | 0,5 - 4 | - | 0,2 - 1 | 0,1 | 0,1 - 0,2 | - |
| Stickstoff N etwa | 71 | 77 | 74 | 77 | 76 | 77 |
| Abgastemperatur °C | 270 | 170 - 220 | 500 - 600 | 300 - 400 | 750 - 850 | 600 - 700 |

Bei der Verbrennung der Kohlenwasserstoffe des Kraftstoffs mit Luft bleiben in der Hauptsache Kohlendioxyd, Wasserdampf und der in der Luft enthaltene Stickstoff übrig. Die unverbrannten Abgasbestandteile Sauerstoff, Wasserstoff, Kohlenoxyd, Methan sind ein Maß für die Güte der Verbrennung. Bei schlechter Verbrennung bleiben Ruß und Teer übrig. Der Ottomotor arbeitet bei Leerlauf und kleinen Drehzahlen mit Luftmangel und überfettetem Gemisch. Die Abgase enthalten viele unverbrannte Bestandteile. Der Dieselmotor arbeitet von Leerlauf bis Vollast ("Rauchgrenze") mit Luftüberschuß. Die Abgase enthalten deshalb nur Spuren von unverbranntem Kraftstoff.

**Kohlenoxyd**. Das farb- und geruchlose Kohlenoxydgas ist sehr giftig! 0,3% in der Atemluft können in 30 Minuten tödlich wirken! 0,03% können noch Gefahr bringen, weniger als 0,01 % sind meist nicht mehr gefährlich Deshalb - Motor nicht in geschlossenem Raum laufen lassen, besonders, wenn Auspuff nicht in Richtung auf die geöffnete Tür geht! - Raum gut entlüften!

### Temperatur und Energie der Abgase

Die Temperatur der Abgase hängt vom Grad der Gas-Entspannung beim Arbeitshub ab. Motoren mit höherem Verdichtungsverhältnis liefern deshalb weniger heiße Abgase (bei Dieselmotoren im Mittel 500°C, bei Ottomotoren 800°C). Die in den Abgasen als Druck und Wärme gespeicherte Energie (etwa 25 - 30%der Kraftstoff-Energie) wird in Abgas-Turbinen (Drehzahlen ca. 20.000 bis ca. 100.000 U/min) genutzt. Und / oder: Ohne 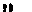 Wärmetauscher" trifft der Abgasstrom direkt auf die zu behandelnden Materialien, so daß diese mit einem Schmelzpunkt bis ca. 600°C verformt bzw. getrennt werden können.

### Kühlmittel für V-Motoren

Neben Flüssigkeiten können auch Gase als Kühlmittel für V-Motoren zweckentsprechend multifunktional verwendet werden. Dies soll möglichst ohne Pumpe / Wärmetauscher, bei Flüssigkeiten durch Zulaufgefälle, erfolgen. Wie zuvor und auf Blatt 1 kurz angesprochen, kann das zusätzlich durch die heißen Abgase erzeugbare Gas-Dampf-Gemisch in mechanische Energie, zum Beispiel durch eine Rohrturbine, umgewandelt werden. Möglich ist ebenso eine Gas- / Dampf-Verwertung durch eine Gas-/Dampf-Kolbenmaschine bzw. Turbine. Aus Wasserdampf lassen sich anschließend Brauchwasser, und aus Meerwasser Frischwasser und Salze gewinnen. Selbst für den Betrieb von Schwimmbädern, etc. eignet sich dieses Verfahren, denn das Wasser wird erhitzt, entkeimt - falls erforderlich zusätzlich durch hohen Druck, indem es auf eine Prallwand gesprüht und ohne Chemikalien aufbereitet wird. Kühlmittel können zum Beispiel Abwässer sein, die vorzüglich als Hochdruckstrahler (z.B. Dampf-, Heißwasser- Gasgemisch mit / ohne abrasive Zusätze) einsetzbar sind. Grundsätzlich gilt, wenn verfahrensbedingt Temperaturerhöhungen erforderlich sein sollten, kann dies durch Umwandlung von mechanischer Energie oder Solarenergie geschehen. Falls zweckmäßig, werden zum Beispiel die Frischgase bzw. Flüssigkraftstoffe mit / ohne Verbrennungsluft für den V-Motor durch die Kühlkanäle gefuhrt.

**Schallwellenenergie** und Abgase von Kolbenmotoren werden vom Auslaßkanal zum Beispiel direkt in den Materialbehälter geleitet. Die hochfrequente Schallwellenenergie kann motortechnisch u.a. durch die Anzahl der Zylinder sowie Zündfolge beeinflußt werden. Schallwellen von Kolbenmotoren entstehen während der Verbrennung des verdichteten Kraftstoff-Luft-Gemisches durch komplexe, hochfrequent aufeinanderfolgende Kompressions- und Dekompressionsphasen. Es entstehen hierdurch intensive Gasschwingungen, die durch die Abgase in mechanische Schwingungen umgewandelt werden. Dies bewirkt einerseits das Entstehen von mikroskopisch kleinen Bläschen und andererseits schlagartiges Zusammenfallen dieser Bläschen aufgrund der extremen Gasverdichtung. So wirken energiereiche Turbulenzen bis in kleinste Hohlräume an den zu behandelnden Teilen bzw. Materialien. Besonders effektvoll ist dies bei der Sterilisation bzw. Keimtötung von Schüttgut (Klinikmüll). Ultraschall ist aufgrund des Kavitationseffektes eine vorzügliche Alternative zum Reinigen sowie Sterilisieren / Desinfizieren von Gegenständen und diversen Materialien, zum Beispiel kontaminierte Filter.

**Elektrische Energie** kann den beschriebenen Stoffbehandlungsprozeß teilweise optimal gestalten bzw. unterstützen. Nachstehende Anwendungsmöglichkeiten sind in Kombination mit den Vorteilen von V-Motoren bestens geeignet, evtl. Lücken zu schließen und den Wirkungsgrad des Verfahrens zu erhöhen; zum Beispiel durch: · Mikrowellen · Stark-/Schwachstrom · Elektro-chemische Verfahren · Widerstandserwärmung, direkt /indirekt · Infrarotwellen

### Mikrowellenenergie

Mikrowellen gehören zur Familie der elektromagnetischen Wellen und sind verwandt beispielsweise mit Radio- und Fernsehwellen, dem sichtbaren Licht und den Röntgenstrahlen. Sie unterscheiden sich nur in der Wellenlange Mikrowellengeräte für den Privathaushalt arbeiten mit einer Frequenz (Schwingungszahl) von 2.450 Megahertz und einer Wellenlänge von 12,25 Zentimetern. Das entspricht fast 2,5 Milliarden Schwingungen in der Sekunde. Beim Kochen dringen die in einer Vakuumröhre (Magnetron) erzeugten und in den Garraum gesendeten schnellen Wellen direkt in die Speisen ein und bringen dort die Wasser-, Fett- und Zuckermoleküle auf Trab. In aberwitziger Geschwindigkeit schwingen diese hin und her, reiben sich dabei aneinander und erzeugen so die nötigen Hitzegrade im Lebensmittel. Das passiert meist wesentlich schneller als bei der Wärmeübertragung im Backofen oder auf der Herdplatte. Erhitzen können die Mikrowellen nur Stoffe, die genügend polarisierte und freibewegliche Moleküle haben. Besonders die zweipoligen Wassermoleküle reagieren heftig im elektromagnetischen Feld. ***Stark wasserhaltige Stoffe eignen sich deshalb besonders gut zur Desinfektion / Sterilisation durch oder mit Hilfe von Mikrowellenenergie. Begünstigt wird dies auch dadurch, daß die Abgase von V-Motoren Wasserdampf enthalten und Flüssigkeit verdampfen, somit das zu behandelnde Material mit Wasserdampfdruck benetzen bzw. durchdringen.***

Mit dem zusätzlichen Einsatz der Mikrowellentechnik können teilweise bessere Verfahrensergebnisse erzielt werden.

**Strom** als Reagenz kann zum Beispiel Problemabfälle vernichten, Keime töten und Abwässer teilweite reinigen. Wo der Strom als Reagenz fungiert, sind es Elektronen, die Substanzen reduzieren oder oxidieren und auf diese Weise auch chemisch sehr stabile Moleküle knacken. Elektrisch geladene Teilchen wirken wie ein Waschmittel, indem sie mit schädlichen Molekülen reagieren und sie beseitigen.

Elektrochemische Verfahren sind zwar grundsätzlich bekannt, sie müssen aber für die relevanten Zwecke verbessert und mit den vorgenannten Techniken kombiniert werden, um optimale ökonomische und ökologische Ergebnisse zu erzielen. Normalerweise sind solche Anlagen leicht zu bedienen und deshalb eine Alternative für Unternehmen, die diverse geringe Abfallmengen entsorgen. Solche gekoppelte Anlagen und Verfahren können bisher eingesetzte Chemikalien überflüssig machen.

Auch stark salzhaltige Abwässer aus der chemischen Produktion, die oft toxische und schwer abbaubare organische Verbindungen in geringen Konzentrationen enthalten, lassen sich ergänzend mit den vorgenannten Verfahren elektrochemisch reinigen; Aktivkohlefilter sind dann nicht mehr notwendig.

**Elektrokinetik** setzt Gifte in Bewegung. Selbst Porenwasser mit den darin gelösten Schadstoffen kann positiv behandelt werden. Eine elektrokinetische Methode kombiniert mit V-Motoren (Saugen + Elektroturbolader) könnte die Reinigung durch Aktivkohlefilter überflüssig machen. So zum Beispiel beim Abbau der Chromatbelastung. Die elektroosmotischen Prozesse, die Bewegung einer Flüssigkeit in Relation zu einer geladenen Oberfläche, ermöglichen den Austrag in der Porenlösung dispergierter bzw. gelöster organischer Moleküle. Ein elektrisches Feld im Gasbad (der Gasstrom wird über Düsen durch Flüssigkeiten geführt) ermöglicht eine weitere Stofftrennung.

### Weitere Anwendungsbeispiele:

**Altbatterien** für den Haushalt lassen sich im heißen Abgasstrom zerlegen; die quecksilberhaltigen Bestandteile werden vergast, das Gas tritt zum Beispiel am Boden eines Flüssigkeitsbeckens nach unten aus (Gasdusche), das Hg lagert sich am Boden eines Wasserbeckens ab.
In Krankenhäusern werden **Quecksilberverbindungen** als desinfizierende Zusätze verwendet, die Pharmaindustrie nutzt sie als Konservierungsmittel, die bedeutendste Quelle ist jedoch die Chlor-Alkali-Elektrolyse nach dem Amalgamverfahren zur Chlorherstellung, einem wichtigen Grundstoff der Chemie. Bei diesem Prozeß fallen Abwässer an, die giftige Hg-Verbindungen enthalten.

### Bioabfälle vergasen.

Wie auf Blatt 3 beschrieben, lassen sich mit dem neuen Verfahren Biomaterial bzw. -abfälle kostengünstiger, schneller und hygienischer verwerten als bisher. Die effektivere Umwandlung des Materials in Gas kann durch Hitze- und Druckregelung erfolgen. Flüssigkeiten können bei Bedarf separat und leichter vergärt werden. Falls erforderlich wird das Material durch einen Quetschvorgang zerkleinert, Feststoffe und Flüssigkeiten getrennt (siehe Blatt 6). Weil Schwermetalle und andere Schadstoffe sich überwiegend in Feststoffen speichern, lassen sie sich aus deren Asche leicht eliminieren. Vorteilhaft ist ferner, daß zum Beispiel Verpackungen nicht stören - sortieren entfällt - weil die Feststoffe entfeuchtet und zu Festbrennstoffen verformbar sind.

Falls erforderlich, trennen Magnete den eisenhaltigen Schrott vom Restmüll. Stör- und Kunststoffe sowie Textilien und Glas (ca. 40%) werden separiert. Nach der TA-Siedlungsabfall dürfen kalte Verfahren mit anschließender Deponierung nur noch bis zum Jahre 2005 angewendet werden.

### Bodensanierung / Altlasten

Der V-Motor, zum Beispiel eines Spezial-Baggers saugt die belastete Bodenluft während des Ausbaggerns des kontaminierten Bodens ab und verwertet sie im (eigenen) V-Motor energetisch.

Um die Freisetzung von leichtflüchtigen Kohlenwasserstoffen aus belastetem Erdreich während der Sanierung zu verhindern, errichtet man üblicherweise über dem Gelände eine luftdichte Halle, deren Abluft dann ständig gefiltert werden muß. Bauarbeiter dürfen sich oft nur in Schutzanzügen und mit Atemschutzmasken bewegen, und sogar die klimatisierte Kabine des Baggerfahrers muß gefiltert werden.

**Neuheit:** Über der Baggerschaufel wird die Luft durch den Saugmotor, bei Bedarf zusätzlich durch einen E-Turbolader abgesaugt, gefiltert und energetisch verwertet. Teuere Aktivkohlefilter sind nicht mehr erforderlich. Die Absaugung kann auch so erfolgen, daß sich zwischen zwei Unterdruckzonen ein Wirbel bildet und somit örtlich eine erhöhte Luftgeschwindigkeit auftritt, um Stäube aufzunehmen. In der Holzbranche (BG-Holz) wurden mit diesem Effekt die Staubemissionen sehr stark reduziert.

### Desinfizieren/Sterilisieren

· Samen · Blumenerde · Klärschlämme · Schuhe, gebraucht · Sachen · leere Gebäude · Klinikmüll · Lederhäute

### Eindampfen

Durch Eindampfkristallisation können Abfallsäuren selektiv, zum Beispiel in saubere Salzsäure, Eisenchlorid und Restverschmutzungen sowie Dampf getrennt und verwertet werden. Salzsäure ist zum Beispiel in einem Galvanik-Betrieb wiederverwertbar. Abgetrenntes Eisenchlorid eignet sich zur Phosphatfällung in Abwässer. Somit ist eine Abfallminderung bis zu ca. 90% möglich. Infolge von eingesparter Entsorgungskosten, verringertem Verbrauch von Salzsäure, Verwertung von Dampf und Eisenchlorid, ist das Verfahren als 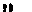 bestens" zu bewerten.

### Erzeugen Chemierohstoffe

Wie zuvor beschrieben, können Kunststoff- und Gummiabfälle in einer NOₓ/O₂-Atmosphäre (Abgase von V-Motoren) durch ein temperatur- und druckgeregeltes Verfahren in wertvolle Chemierohstoffe umgewandelt werden. Aus PE und Polystyrol entstehen organische Verbindungen wie Benzoe- Glutar- und Bernsteinsäure. Diese sind als hochwertige und teure Ausgangsstoffe für weitere organische Synthesen für die chemische Industrie interessant. Aus PE lassen sich sogar komplizierte Alpha-Omega-Disäuren, die für die Herstellung technischer Polymere und Fasern geeignet sind, erzeugen. Styropor-Abfälle, die zu ca. 98% aus Luft bestehen, bilden zusammen mit den teilweise gebundenen Abgasbestandteilen eine neue chemische Verbindung, ähnlich wie Aceton. Biodiesel und diverse Fettsäuren, die Kunststofftanks und Gummi 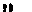 auffressen" sättigen sich mit Abgasbestandteilen. Wie Versuche zeigten, scheint es möglich, Biodiesel durch die Anreicherung mit Abgasbestandteilen zu Reinigungsmitteln zu veredeln. Es wird angenommen, daß 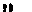 wertloses" und klimaschädliches CO₂ in eine wertvolle chemische Verbindung umwandelbar ist (siehe hierzu angewandte Chemie Band 111, S. 373, 99). Sollte dieses Verfahren auch bei Kraftfahrzeugen mit V-Motoren anwendbar sein, so könnte die CO₂-Problematik entschärft werden. Unter anderem kann auch N₂ sinnvoll separiert und gespeichert werden, da der Luftstickstoff als Nebenprodukt anfällt. Das Verfahren nutzt die Fähigkeit von Kraft- und Kunststoffen, (Schad)stoffe aufzunehmen, die in 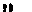 technische Gase" umgewandelt werden können. Schließlich demonstriert das Verfahren emissionsarme Technik. Es trägt neben dem Wegfall von Entsorgungskosten auch zur Ressourcenschonung fossiler Brennstoffe und zur Minderung energiebedingter CO₂-Emissionen bei.

Die bekannten Pyrolyseverfahren sind unrentabel, denn die Energie für nötige hohe Prozeßtemperaturen ist zu teuer. Wenn im Reaktor als Wärmeträger Zeolithe anstelle Quarzsand als Reaktionsbeschleuniger verwendet werden, kann die Stoffumwandlung schon bei niedrigeren Temperaturen funktionieren.

**Gase** absaugen aus: · Räumen, zum Beispiel Lösemittel · Arbeitsstätten · Boden, kontaminiert · Biogasanlagen · Klärschlämme · Abwässer · Abluftanlagen · Jauchegruben · Papierindustrie · Tierkörperbeseitigungsanlagen und energetisch verwerten.

**Klärschlämme und Abwässer** reinigen bzw. aufbereiten. Im Zusammenwirken der heißen, druckvollen Abgase von V-Motoren und deren Schallwellenenergie (Ultraschall) sowie mechanischer Energie wird der bekannte Prozeß weiter verbessert; es bilden sich sogenannte Kavitationsblasen mit großen Scherkräften, die Zellstrukturen gezielt zerstören können. Gleichzeitig finden auch chemische Reaktionen in den Kavitationsblasen, bei 5.000 Grad Kelvin und 500 bar, statt. Dadurch wird der in den Bläschen enthaltene Wasserdampf in sehr reaktive Wasserstoff- und Hydroxyl-Radikale zerlegt. Sie gehen sowohl in der Blase wie im umgebenden Medium neue Verbindungen ein. Auf diese Weise können halogenhaltige Schadstoffe dehalogeniert und in biologisch abbaubare Verbindungen zersetzt werden. Insbesondere in Deponiesickerwässern und industriellen Abwässern finden sich schwer abbaubare Inhaltsstoffe, die aufgrund ihrer geringen Wasserlöslichkeit nur sehr langsam biologisch abgebaut werden können Bisher setzt man vor allem Chemikalien ein, um sie für die nachfolgende biologische Behandlung aufzuschließen.

Erfindungsgemäß sollen die heißen, druckvollen Abgase und Schallwellen von V-Motoren direkt in den Klärschlamm bzw. in das Abwasser gezielt geleitet werden, wodurch der Klärschlamm schneller fault und die Mikroorganismen abtötet. Analog kann dieser Prozeß auch zur Abwasserbehandlung angewendet werden Ultraschall kann überall helfen, wo Kläranlagen an ihre Kapazitätsgrenze stoßen. Damit läßt sich die Schlamm-Menge erheblich reduzieren. Versuche haben ergeben, daß hochfrequente Schallwellen industrielle Abwässer entgiften können. In Flüssigkeiten erzeugen die Schallschwingungen kleine Gasbläschen. Diese Bläschen vergrößern sich und fallen schließlich in sich zusammen. Dabei entstehen auf kleinem Raum sehr schnelle Strömungen, die auf feste Oberflächen ähnlich wie Hochdruckstrahler wirken. Diese Strömungen führen aber auch dazu, daß Mikroorganismen oder Klärschlämme mechanische behandelt werden und den biologischen Abbauvorgang beim Klärschlamm beschleunigen. Die neuen Anlagen müssen den Schlamm nicht mehr mechanisch zerkleinern. Das verringert unter anderem den Verschleiß der eingesetzten Geräte. Die Spaltprodukte sind nach der Behandlung nicht mehr giftig und lassen sich leichter biologisch abbauen. Erfindungsgemäß werden die thermischen, chemischen und mechanischen Kräfte genutzt, die entstehen, wenn ein wäßriges Medium Ultraschall ausgesetzt wird.

In einem Reinigungsbad wird die hochfrequente Energie in mechanische Energie umgewandelt. Relevant ist die richtige Auswahl des Reinigungsmediums.

Neben Staub, verkrusteten Fetten, Ölen, Trennmitteln, Farbrückständen etc. gibt es vielfältige Reinigungsaufgaben im Bereich der Dekontamination / Desinfektion / Sterilisation.

Zusätzlich können geeignete Abwässer als Durchlaufkühlmittel für V-Motoren für einen anschließenden Dampfbetrieb(-Antrieb) verwendet werden. Dies kann auch im Kreislauf geschehen, wobei das Abwasser desinfiziert/sterilisiert wird und der Dampf in das Abwasser geleitet und kondensiert wird. Ebenso Schwimmbäder: Wasser erhitzen, sterilisieren und reinigen im Kreislauf - Meerwasserentsalzung → dort.

### Recycling

Die Entsorgung von PVC-beschichteten Holzwerkstoffen bereitet keine Probleme, da eine moderne Rauchgasreinigung möglich ist. Wie Messungen diverser Institute belegen, zeigen Elektrofilter bezüglich giftiger Chlorverbindungen keine Abscheidwirkung.

### Aluminium

Alu-Abfälle in Verbund mit Kunststoffen, Papier, Lacke etc. lassen sich bisher auf Grund schädlicher Rauchgase und O₂ schlecht einschmelzen. In einer O₂-mageren Abgasatmosphäre von V-Motoren treten diese Probleme nicht auf. Das erzeugte Pyrolysegas wird in V-Motoren energetisch verwertet. Gleichzeitig kann der Alu-Abfall gereinigt, und zum Beispiel zu Pellets verdichtet oder einer Schmelzanlage direkt zugeführt werden.
**Lederabfälle** → Mischwerkstoffe, zum Beispiel Schuhsohlen

### Sondermüll

| | | |
|---|---|---|
| - desinfizieren /sterilisieren | - (BSE)) inaktivieren | |
| - dekontaminieren | Temperatur | ≥ 133°C |
| (Hg, Blei, Cadmium .....) | Druck | ≥ 3 bar |
| | Prozeßdauer | ≥ 20 Minuten |

### Trennen

Schleifschlämme von Öl, Wasser, Metall- und Glasspänen. Die heißen, druckvollen Abgase - unterstützt durch die Schallwellen - von V-Motoren werden direkt in die Schleifschlämme geleitet, wodurch sich der Schlamm erhitzt. Folge: Wasser verdampft, das heiße Öl wird durch ein Sieb gedrückt und gesammelt. Der Schlamm wird pulverisiert und kann (heiß)-isostatisch zu Produkten geformt werden. Es erscheint möglich, Abgasbestandteile zu integrieren und dadurch den Recyclingprozeß zu optimieren.

Somit lassen sich aus den Schleifschlämmen, zum Beispiel metallverarbeitender Betriebe, sowohl Öl als auch Metalle wiederverwerten. Bei der Bearbeitung von Metallen werden in Deutschland jedes Jahr ca. 2 Milliarden Liter Kühlschmierstoffe verwendet. Ca. 30% dieser auf Erdölbasis hergestellten Produkte verflüchtigen sich bei ihrem Einsatz und können gesundheitliche Schäden verursachen. Während des Verfahrensprozesses werden die Gase abgesaugt und verwertet, gegebenenfalls durch den V-Motor.

### Vergasen mit / ohne O₂-Mangel

· Altlacke · Fette · Altöle · Schleif-/Schmieröle · Emulsionen · Speise-/Lebensmittelreste · Holz · Kunststoffabfälle · Gülle/Mist · Lederabfälle · Bio-Masse (Pflanzen). Das Verfahren kann infolge der hohen Abgastemperaturen und Drücke sowie Schallwellen ein Gasgemisch aus ca. 80% H₂ und ca. 20% Methan erzeugen.

### Wasseraufbereitung - Meerwasser

**Meerwasser dient als Kühlmittel für V-Motoren, Frischwasser und Salze werden dabei gewonnen, ggf. kombiniert wie nachstehend beschrieben. Gleichzeitig kann die mech. und weitere thermische Energie des V-Motors zwecks Wasserbehandlung oder für andere Zwecke genutzt werden.**

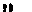 Bereits die Hälfte der weltweit etwa 12.500 km³ nutzbaren Wassers wird heute schon genutzt. Die Menschheit verbraucht heute sechsmal soviel Wasser wie zu Beginn des 20. Jahrhunderts, der Verbrauch pro Kopf hat sich verdoppelt. In vielen Gegenden der Welt wird das Wasser immer knapper. Wissenschaftler erwarten im kommenden Jahrhundert die ersten Kriege um Trinkwasser. Schon heute streiten Israel und seine Nachbarn um das Jordanwasser. Die Türkei will den Euphrat stauen und damit dem Irak und Syrien das Wasser abdrehen.

Die Bedeutung der Meerwasserentsalzung nimmt daher künftig rapide zu. Noch allerdings ist das Verfahren sehr energieintensiv und teuer. Nach Angaben der Salzgitter Anlagenbau AG, einem der größten Hersteller von Meerwasserentsalzungsanlagen in Deutschland kostet eine solche Anlage zwischen 1.200 Dollar und 1.500 Dollar pro Kubikmeter installierter Leistung.
Derzeit konkurrieren noch viele Verfahren zur Meerwasserentsalzung miteinander, so zum Beispiel die
- Entspannungsverdampfung (Multi Stage Flash Evaporation, MSF). Sie ist das gebräuchlichste Verfahren. Dabei wird das Salzwasser auf 90 - 130°C erhitzt und durch bis zu 30 Kammern geleitet. In jeder dieser Kammern herrscht ein niedrigerer Druck als in der vorhergehenden. Das Wasser verdampft also in der zweiten Kammer schneller als in der ersten und so weiter. Der Wasserdampf wird aufgefangen.
- Destillation: Das Salzwasser wird in einem Behälter so lange erhitzt, bis das Wasser verdampft ist. Der Wasserdampf wird aufgefangen und wieder abgekühlt. Findet die Destillation in einem Vakuum statt, verdampft das Wasser bei niedrigerer Temperatur. So spart der Betreiber der Anlage Heizkosten. An manchen Standorten, zum Beispiel lauf Hawaii, wird das erhitzte Wasser zur Stromgewinnung und kaltes aus dem Ozean zur Kondensation genutzt:
- Elektrodialyse: Die positiv und negativ geladenen Salzionen werden durch eine poröse Membran herausgefiltert;
- Ionenaustauscher: Wasser wird über eine Kunststoffschicht geleitet, die der Salzlösung die Salzionen entzieht;
- Tiefkühlen: Das Wasser stößt das Salz ab, wenn es gefriert;
- Umkehrosmose: Der vom Salzwasser ausgehende natürliche Druck drängt das Wasser durch eine Membran, die keine anderen Stoffe durchläßt. Salz bleibt zurück.

Da alle Verfahren zur Entsalzung des Meerwassers sehr viel Energie verbrauchen, experimentieren viele Hersteller und Institute mit solarenergiebetriebenen Entsalzungsanlagen. Das Fraunhofer Institut für Solare Energiesysteme in Freiburg betreibt mit Förderung der Europäischen Union eine Versuchsanlage auf den Kanarischen Inseln. Dort setzt das Institut erstmals statt der Sonnenwärme-Absorber aus Metall, die im Salzwasser schnell rosten, neuentwickelte Kunststoffabsorber ein" (VDI).

Die **mobile / stationäre Anlage** zur Stofftrennung und -behandlung, Sterilisation von kontaminierten Materialien sowie eine rationelle Gasgewinnung und deren wirtschaftliche Verwertung besteht hauptsachlich aus - im Flußprinzip verknüpft - folgenden Varianten:
1) Fahrgestell bzw. Gestell
2) V-Motor
3) Abgasturbine (Rohrturbine)*
4) E-Turbolader
5) Stromaggregat
6) Material-Behandlungsbehälter, mit / ohne Bestrahlungsstation*
7) Behälter* für behandeltes Material
8) Material-Quetscher und -Förderer*, mit / ohne elektr. Keimtötung
9) Schneid- und Förderschnecke*, mit / ohne elektr. Keimtötung
10) Flüssigkeitsbehälter* (Gasbadewanne), mit / ohne energetischer Wirkung
11) Gas-Mat-Leitungsrohr, mit / ohne integrierter Schneid- / Förderschnecke*
12) Wirbelstromabscheider*
13) Mikrowellenstation*
14) Drehrohr (Siebe)*
15) Magnetabscheider*
16) Gasabscheider* und -Aufbereiter
17) Zweiter V-Motor* (* = Variante).

Je nach Anwendungsfall kann die Anlage kontinuierlich als auch diskontinulierlich arbeiten.

**Beispiel**: Die mobile Anlage kann alle gängigen Gebinde zum Beispiel von Klinik-Sondermüll bei jeder Wetterlage und dynamischer Belastung - auch während des Transportes im Straßenverkehr - behandeln. Im Grundprinzip funktioniert dies so wie bei einem Lkw-Betonmischer, wobei - falls erforderlich - der Sondermüll durch eine Zahnradwalzenstation zerquetscht - bei Bedarf durch elektrische Energie inaktiviert - und in die Trommel gefördert wird. Zusätzlich werden die heißen keimtötenden Abgase und Schallwellen vom V-Motor auf kürzestem Wege direkt oder durch eine Gasturbine in die Trommel gedrückt. Die Trommel dient als Behandlungs- und Transportbehälter. Aus der Trommel werden die durch die Verschwelung entstandenen Gase durch den V-Motor oder Turbolader aller Art, zum Beispiel E-Turbo, abgesaugt und zum Beispiel im V-Motor energetisch verwertet. Im Durchschnitt reduziert sich das Feststoffvolumen von 100% auf unter 10%, das nach der Inaktivierung weiter verwendet werden kann, jedenfalls kein Sondermüll mehr ist.

Die Abgas-Rohrturbine besteht aus dem Abgasrohr, das in ein Anschlußgasrohr (Rohrstutzen) mündet bzw. hineinragt, dessen Innendurchmesser > Außendurchmesser des Abgasrohres ist und der Rohrstutzen so geformt ist, daß der volle Abgasstrom den Rohrstutzen mit hohen Drehzahlen antreibt, das heißt das erste Rohr bildet die Drehachse des Rohrstutzens. Wenn der Rohrstutzen bzw. Rohrschlange richtig gekrümmt ist (große Gasumlenkungswinkel, Umfangskomponenten der Strömungsgeschwindigkeiten groß), wird die Schaufelarbeit-Schnittleistung optimiert, das heißt hohe Drehzahlen = große Absolutgeschwindigkeiten und auch bei kleinen Gasdurchsätzen werden gute Wirkungsgrade erzielt.

Am Rohrstutzen befestigte Werkzeugschneiden ermöglichen einen sinnvollen Unwuchtausgleich (siehe auch Blatt 5 Absatz 3).

Gelagert wird die Turbine vorzugsweise durch den Abgasdruck und / oder Kohlenstoff-Geitlager, wobei die Schmierung durch die Abgase erfolgt. Die Abgasturbine kann von vertikal bis horizontal angeordnet werden.

Bei einer anderen Variante kann das Fahrgestell mit Luftfederung fast bis zum Boden abgesenkt werden um den Arbeitsablauf zu erleichtern.

Vorzugsweise zeichnet sich der V-Motor durch Keramik-Kolbenlaufflächen, -Ventile, -Zylinderköpfe, -Abgaseinzelrohre (isoliert), -Einspritzdüsen und Kohlenstoffkolben aus.

Der **Material-Quetscher** und -**förderer** wirkt ähnlich wie eine Zahnradpumpe. Bei Überlast vergrößern sich automatisch die Achsenabstände der Zahnradwalze (n), um Schäden zu vermeiden. Hier eignen sich u.a. Gummilagerungen, die auch als Stromisolator fungieren. Falls zweckmäßig, werden Keime durch eine Stromwiderstandserhitzung sterilisiert, die noch nicht durch Druck, Reibung, Starkstrom, Ozon und eventuell Motorabgase abgetötet wurden.

Der **Wirbelstromabscheider** bzw. das Wirbelstromrohr kann zwecks Optimierung und Anpassung des Verfahrensprozesses dauermagnetisch sein bzw. elektrische und magnetische Felder aufbauen. Für besondere Anforderungen ist ein Katalysator nachschaltbar.

## Patentansprüche

1. **Verfahren zur Stofftrennung /-behandlung,Sterilisation, auch von kontaminierten Materialien und / oder Frischgasgewinnung, sowie deren wirtschaftliche Verwertung, insbesondere durch heiße Gase von Verbrennungsmotoren oder Gasturbinen (V-Motoren), dadurch gekennzeichnet, daß**
1.1 fast die gesamte mechanische als auch thermische Nutzenergie der V-Motoren einschließlich deren Schallwellen und keimtötenden Abgase sowie Kühlmittel zur Stofftrennung, Sterilisation und / oder Frischgasgewinnung optimal verwertet werden, indem die heißen, keimtötenden, druckvollen Abgase und Schallwellen - bei Bedarf zusätzlich Strahlungsenergie in Form von Heißluft und / oder Heiß-Wasser-Dampf mit hoher Geschwindigkeit direkt, oder nach Durchströmung einer Rohrturbine, auf Gegenstände / Stoffe wirken, Stoffe behandeln, sterilisieren, vergasen, trennen; die wertvollen Ab- und Frischgase aufbereitet, vorwiegend im ersten und / oder zweiten V-Motor umweltfreundlich, energetisch verwertet oder anderweitig genutzt werden; ergänzend die mechanische Energie zum Antrieb der mobilen Anlage und von mechanischen Vorrichtungen sowie zur Erzeugung von Über-/ Unterdruck, Strom- und Druckluft dient; bei Bedarf zusätzlich eine Gas-Material-Nachbehandlung mit Mikrowellen oder Bestrahlungen oder Elektrospannung vorgesehen ist;
1.2 der Verfahrensprozeß zwecks Vorbehandlung separat, oder kombiniert einleitend bzw. nachgeschaltet, erfolgt durch mechanischen Druck und Reibung sowie Elektrospannung, indem die zu behandelnden Stoffe zwischen Zahnrädern Schraubenwalzen, Schnecken, Extrudern, Schneidwerkzeugen gequetscht, zerrieben, zerkleinert, sterilisiert und transportiert werden, bzw. der Strom von Walze zu Walze durch das Material geleitet wird,
1.3 Flüssigkeiten bzw. Gase als Durchlaufkühlmittel für V-Motoren, erhitzt, entkeimt, vergast, verdampft und zweckentsprechend - auch im Kreislauf - aufbereitet werden und multifunktional dienen.

2. Verfahren (V) nach (n) Anspruch (A) 1.1, 1.3, dadurch gekennzeichnet, daß die Flüssigkeiten Abwässer / Gasgemische sein können, wobei der Dampf und evtl. die Motorabgase auch nach einem Gas-/Dampfbetrieb - ggf. im Kreislauf - in die Abwässer geleitet und kondensiert werden oder der Wasserdampf in Brauchwasser umgewandelt wird.

3. V n A 1.1, 1.3,
dieses Verfahren sich auch für die Meerwasserentsalzung oder für den Betrieb von Schwimmbädern etc. eignet, wenn nötig, das heiße Wasser mit hohem Druck auf eine Prallwand gesprüht und ohne Chemikalien aufbereitet wird.

4. V n A 1.1 - 1.2,
der durch eine Rohrturbine gereinigte Abgasstrom und die Schallwellen direkt auf die zu behandelnden Materialien treffen und diese mit einem Schmelzpunkt bis ca. 600°C verformen bzw. trennen.

5. V n A 1.1,
die heißen, druckvollen Abgase und Schallwellen von V-Motoren direkt in den Klärschlamm bzw. in das Abwasser gezielt geleitet werden, wodurch der Klärschlamm schneller fault, die Mikroorganismen abtötet sowie die Schlamm-Menge erheblich reduziert.

6. V n A 1 - 1.2,
die Gase durch einen Wirbelsromabscheider geführt, getrennt, gereinigt werden sowie mit E-Turbo im Wirbelrohr Unterdruck erzeugt wird, der zur Kälteerzeugung dienen kann.

7. V n A 1 - 1.3,
alternativ die Gase durch V-Motoren (Saugmotoren) auch direkt abgesaugt und (energetisch) verwertet werden.

8. V n A 1 - 7,
Stoffe behandelt, getrennt, abgesaugt, entfernt, verformt, vergast, entgast, dekontaminiert, desinfiziert, zerkleinert, sterilisiert, gefördert, verdichtet, recyclet, energetisch verbrannt, getrocknet, eingedampft, verdampft, entwässert, entfettet, entfrostet, erhitzt
und dabei, je nach Zweck in einer NOₓ / O₂-Atmosphäre, Brenn-/Kraftstoffe, technische Gase, Chemierohstoffe, Dünger, Hitze, Kälte, Dampf, Sattdampf, Heiß-/Warmwasser und Hochdruckstrahlen (Gas und Heiß-/Warmwasser sowie Dampf) erzeugt werden können.

9. Anlage zwecks Realisierung des Verfahrens nach Anspruch 1 bis 1.3, dadurch gekennzeichnet, daß
auf einem Fahrgestell bzw. Gestell, im Gas- bzw. Material-Flußprinzip miteinander verknüft sind: V-Motor, Abgasrohr (e), Rohrturbine* Materialbehandlungsbehälter, mit / ohne Bestrahlungsstation*; Material-Quetscher und Förderer*, mit / ohne elektrische Keimtötung; Drehrohrsieb / Magnetabscheider*, Gas- Material-Leitungsrohr, mit / ohne integrierter Schneid- und Förderschnecke* sowie elektrischer Keimtötung; Behälter* für behandeltes Material und Gas, Wirbelstromabscheider*, Gasbadewanne*, mit /ohne energetischer Wirkung; Gasabscheider*, Gasaufbereiter; 1. V-Motor oder 2. V-Motor* (* = Variante); optimiert durch E-Turbolader / Gebläse, Druckluft.

10. A n A 9, dadurch gekennzeichnet, daß
die Rohrturbine besteht aus dem Abgasrohr, das in ein Anschlußgasrohr (Rohrstutzen) mündet bzw. hineinragt, dessen Innen-⌀ > Aussen-⌀ des Abgasrohres ist; und der Rohrstutzen so geformt ist, daß der Abgasstrom den Rohrstutzen mit hohen Drehzahlen antreibt, d.h. das 1. Abgasrohr bildet die Drehachse des 2. Rohres (Rohrstutzen) und an der Rohrturbine Schneidwerkzeuge angebracht sind.
